# EUROPÄISCHE PATENTSCHRIFT

(11) **EP 3 576 681 B1**
(45) Veröffentlichungstag und Bekanntmachung des Hinweises auf die Patenterteilung: **19.07.2023**
(21) Anmeldenummer: 18703283.4
(22) Anmeldetag: 31.01.2018
(51) Int. Cl.: A61F 2/68, A61F 2/64

(54) **ELEKTROMAGNETISCHES VERRIEGELUNGSELEMENT FÜR EINE GELENK-ORTHESE ODER GELENK-PROTHESE**
ELECTROMAGNETIC LOCKING ELEMENT FOR A JOINT ORTHOSIS OR A JOINT PROSTHESIS
ÉLÉMENT DE VERROUILLAGE ÉLECTROMAGNÉTIQUE CONÇU POUR UNE ORTHÈSE D'ARTICULATION OU UNE PROTHÈSE D'ARTICULATION

(30) Priorität: 01.02.2017 DE 102017000902
(43) Veröffentlichungstag der Anmeldung: 11.12.2019
(73) Patentinhaber: Ottobock SE & Co. KGaA, 37115 Duderstadt (DE)
(72) Erfinder: MECKLENBURG, Arno, 10999 Berlin (DE); ALBRECHT-LAATSCH, Erik, 37124 Rosdorf (DE); NOLTE, Michael, 37136 Seeburg (DE); MÜLLER, André, 37115 Duderstadt (DE)
(74) Vertreter: Gramm, Lins & Partner Patent- und Rechtsanwälte PartGmbB
(86) Internationale Anmeldenummer: PCT/EP2018/052349
(87) Internationale Veröffentlichungsnummer: WO 2018/141770

(56) Entgegenhaltungen:
- DE-A1-102008 059 907
- DE-A1-102010 025 766
- DE-A1-102012 104 173
- US-A- 6 165 226
- US-A1- 2004 225 242
- US-A1- 2007 032 884
- US-A1- 2010 082 115
- US-A1- 2013 253 393

## Beschreibung

Die vorliegende Erfindung betrifft ein elektromagnetisches Verriegelungselement zum Verriegeln des Gelenkes einer Gelenk-Orthese oder Gelenk-Prothese. Gelenk-Orthesen und Gelenk-Prothesen weisen üblicherweise eine erstes und ein zweites Element auf, welche über ein Drehgelenk schwenkbar miteinander verbunden sind. Die vorliegende Erfindung betrifft insbesondere eine Knie-Orthese oder eine Knie-Prothese. Das erste Element ist in diesem Fall am Oberschenkel anordenbar, das zweite Element stützt oder ersetzt den Unterschenkel.

Gelenk-Orthesen werden für Patienten eingesetzt, welche das Kniegelenk nicht oder nicht mehr ausreichend kontrollieren können. Durch die Verriegelbarkeit des Drehgelenkes wird es solchen Patienten ermöglicht, dennoch zu laufen oder zu stehen. Insbesondere kann das Drehgelenk in einer gestreckten Position der Gelenk-Orthese verriegelt werden, so dass die Orthese ein sicheres Stehen oder eine sichere Stütze beim Laufen bietet. In gleicher Art kann eine Gelenk-Prothese einen fehlenden Unterschenkel ersetzen. Eine solche Gelenk-Orthese ist beispielsweise aus der Druckschrift DE103 11 189 B4 bekannt. Die Verriegelung erfolgt hier über einen monostabilen Hubmagneten, welcher einen Stift aufweist, der auf Grund der Schwerkraft im stromlosen Zustand in die verriegelte Position fällt, und durch Anlegen einer Spannung an eine Spule aus der verriegelten Position gefahren werden kann. Diese Konstruktion hat den Vorteil, dass beim Ausfall der Spannungsversorgung automatisch eine Verriegelung erfolgt. Die Konstruktion hat jedoch den Nachteil, dass durch die monostabile Konstruktion ein hoher Stromverbrauch vorliegt. Dies verringert die Strecke, welche ein Patient mit einer solchen Gelenk-Orthese gehen kann, bevor die Akkus der Orthese geladen werden müssen, beträchtlich.

Aus der DE 10 2010 025 766 A1 ist ein die stabile mit einem Gehäuse, einem Permanentmagneten, eine elektrischen Spule und einem entlang einer Bewegungsachse beweglichen Aktor bekannt, wobei der Aktor durch den Permanentmagneten entlang der Bewegungsachse beaufschlagbar ist. Das Gehäuse weist ein Ankergegenstück auf, in dem ein Hilfsgegenstück beweglich angeordnet ist. Der Aktor durchsetzt das bewegliche Hilfsgegenstück und das Gehäuse, das Ankergegenstück und das Hilfsgegenstück sind in einem bestromten Zustand überwiegend durch die Spule magnetisch gepolt. Zwischen dem Aktor und dem Gehäuse ist eine erste Feder angeordnet, zwischen dem Aktor und dem Hilfsgegenstück ist eine zweite Feder angeordnet. In einem unbestromten Zustand der Spule wird die zweite Feder bei einer Festlegung des Aktors durch den Permanentmagneten in einem Bereich gestaucht.

Aus der US 2004/022242 A1 ist ein orthopädietechnisches Hilfsmittel mit einer Verriegelungsvorrichtung bekannt, mit der zwei beweglich zueinander gelagerte Teile in einer vorbestimmten relativen Position verriegelt werden können. Über einen Hubmagneten ist es möglich, ein Verriegelungselement aus der Verriegelungsposition in eine Freigabeposition zu bewegen. Wird der Magnet nicht mehr Strom, fällt das Verriegelungselement nach unten und verriegelt das Gelenk, wenn eine Verriegelungsstellung der beiden Teile zueinander erreicht ist.

Aufgabe der vorliegenden Erfindung ist es daher, ein verbessertes elektromagnetisches Verriegelungselement für eine Gelenk-Orthese oder Gelenk-Prothese zur Verfügung zu stellen.

Diese Aufgabe wird durch ein elektromagnetisches Verriegelungselement gemäß Anspruch 1 gelöst. Vorteilhafte Ausgestaltung der vorliegenden Erfindung sind Gegenstand der Unteransprüche.

Die vorliegende Erfindung ist durch die beigefügten Ansprüche definiert und umfasst ein elektromagnetisches Verriegelungselement zum Stoppen und/oder Vereinzeln von auf einer kontinuierlich betriebenen Stückgut-Förderanlage geförderten Stückgut-Trägern und/oder Stückgut, umfassend:
- einen bistabilen Hubmagneten mit mindestens einer Spule und mindestens einem Permanentmagneten;
- einen Stift, welcher mit Hilfe des Hubmagneten ein- und ausgefahren werden kann,
- einen oder mehrere elektrische Energiespeicher, insbesondere Kondensatoren, eine Steuerung, welche mit Hilfe von Schaltern den oder die Energiespeicher über die mindestens eine Spule des Hubmagneten so entlädt, dass der Stift ausgefahren wird.

Durch den Einsatz eines bistabilen Hubmagneten und die Energiespeicher wird ein sehr viel energiesparenderer Betrieb des Verriegelungselements möglich.

In einer möglichen Ausführungsform der vorliegenden Erfindung weist der bistabile Hubmagnet zwei Hubendlagen auf. Eine erste Hubendlage entspricht bevorzugt der komplett eingefahrenen Position des Stifts, die zweite Hubendlage der komplett ausgefahrenen Position des Stifts. In der komplett ausgefahrenen Position verriegelt der Stift bevorzugt das Gelenk, in der komplett eingefahrenen Position gibt der Stift das Gelenk bevorzugt frei.

Bevorzugt ist ein Federsystem vorgesehen, welches einen oder mehrere Anker des bistabilen Hubmagneten aus den Hubendlagen heraus in Richtung einer Hubmittellage vorspannt. Das Federsystem erhöht die Kraft des Hubmagneten und erlaubt einen energiesparenden Betrieb. Das Federsystem kann in dem Hubmagneten verbaut sein, oder extern zu dem Hubmagneten als ein weiterer Bestandteil des Verriegelungselements ausgeführt sein.

Bevorzugt unterscheidet sich die in den beiden Hubendlagen im Verriegelungselement gespeicherte potentielle Energie ausschließlich der elektrischen Energie und im unbestromten Fall um nicht um mehr als 50% des größeren Wertes voneinander, bevorzugt um nicht mehr als 25%. Für die Berechnung der potentiellen Energie wird die elektrische Energie unberücksichtigt gelassen, und der unbestromte Fall betrachtet. Die potentielle Energie ergibt sich daher zunächst aus der durch die Federn und Permanentmagnete gespeicherten potentiellen Energie. Für den Fall, dass der Stift in vertikaler Richtung bewegt wird, kann bei der Berechnung der potentiellen Energie, welche im Verriegelungselement gespeichert ist, bevorzugt auch die potentiellen Energie des Stifts aufgrund der Schwerkraft berücksichtigt werden. Alternativ kann die potentielle Energie des Stiftes jedoch auch unberücksichtigt bleiben.

In einer möglichen Ausführungsform ist vorgesehen, dass der bistabile Hubmagnet eine asymmetrische Kennlinie aufweist. Insbesondere kann die Kraft und/oder Beschleunigung des Hubmagneten bei einer Einfahrbewegung aus der komplett ausgefahrenen Position des Stifts größer sein als bei einer Einfahrbewegung aus der komplett eingefahrenen Position.

In einer weiteren möglichen Ausführungsform ist vorgesehen, dass wobei die magnetische Haltekraft des Hubmagneten in derjenigen Hubendlage am höchsten ist, in welcher sich der Stift des Verriegelungselements in seiner ausgefahrenen Position befindet.

Bevorzugt wird dies durch eine geometrische Kennlinienbeeinflussung erreicht. Alternativ oder zusätzlich kann die magnetische Haltekraft in einer der Hubendlagen zwischen 20% und 80% der magnetischen Haltekraft in der anderen Hubendlage betragen, bevorzugt zwischen 30% und 70%.

In einer bevorzugten Ausführungsform weist das Verriegelungselement im unbestromten Fall eine Raststelle in einer Position auf, in welcher der Stift teilweise ausgefahren ist. Bevorzugt ist der Stift an der Raststelle hinreichend weit ausgefahren, um das Gelenk zu verriegeln. Hierdurch ergibt sich eine stark erhöhte Sicherheit.

Die Raststelle kann durch eine asymmetrische Kennlinie erreicht werden. Bevorzugt ist die Raststelle gegenüber der Mitte des Hubweges versetzt, wobei die Raststelle insbesondere zwischen der Hubendlage, welche einer komplett ausgefahrenen Position des Stifts entspricht, und der Mitte des Hubweges angeordnet ist.

Der Abstand zwischen der Raststelle und der Mitte des Hubweges beträgt bevorzugt mehr als 5 % des Hubweges, weiter bevorzugt mehr als 10 %, weiter bevorzugt mehr als 20%. Alternativ oder zusätzlich kann der Abstand zwischen der Raststelle und der Hubendlage, welche einer komplett ausgefahrenen Position des Stifts entspricht, bevorzugt mehr als 2 % des Hubweges betragen, weiter bevorzugt mehr als 5 %, weiter bevorzugt mehr als 10 %.

Die Raststelle wird bevorzugt durch eine Raststelle des Hubmagneten zur Verfügung gestellt, insbesondere durch eine dritte, im stromlosen Zustand stabile Hublage des Hubmagneten.

In einer möglichen Ausführungsform ist vorgesehen, dass die Steuerung eine Unterbrechung und/oder ein Abschalten der Spannungsversorgung des Verriegelungselements erkennt und in Reaktion hierauf den Stift ausfährt, wobei bevorzugt das Abfallen der Versorgungsspannung mittels einer Flankenerkennung erkannt wird. Das Verriegelungselement kann daher genauso angesteuert werden wie ein Verriegelungselement mit einem monostabilen Hubmagneten.

Alternativ oder zusätzlich kann die Steuerung so ausgestaltet sein, dass auf ein Zuschalten der Versorgungsspannung hin der oder die elektrischen Energiespeicher, vorzugsweise Kondensatoren, aufgeladen werden, und dass das Erreichen einer bestimmten Schwellspannung am elektrischen Energiespeicher von der Steuerung erkannt wird, woraufhin selbige den oder die Energiespeicher über den bistabilen Hubmagneten so entlädt, dass der Stift eingefahren wird.

In einer möglichen Ausführungsform wird der bistabile Hubmagnet über eine Vollbrücke, insbesondere eine MOSFET-Vollbrücke angesteuert, wobei die Vollbrücke bevorzugt zwei weitere Schalter aufweist, über welche ein erster und einer zweiter Energiespeicher in einem ersten Schaltzustand parallel geschaltet und in einem zweiten Schaltzustand separat entladen werden können.

In einer möglichen Ausführungsform weist die Steuerung mindestens einen ersten und einen zweiten elektrischen Energiespeicher auf, wobei der erste Energiespeicher in Serie über zwei Spulen des Hubmagneten entladbar ist, und wobei der zweite Energiespeicher über nur eine der zwei Spulen des Hubmagneten entladbar ist.

In einer möglichen Ausführungsform ist der zweite Energiespeicher wahlweise über eine der beiden Spulen entladbar. Insbesondere kann die Entladung dabei je nach Bewegungsrichtung über die erste oder die zweite Spule erfolgen.

Alternativ oder zusätzlich kann der zweite Energiespeicher wahlweise auch in Serie über die zwei Spulen des Hubmagneten entladbar sein. Insbesondere kann die Entladung dabei je nach Bewegungsrichtung über eine der beiden Spulen oder in Serie über die zwei Spulen erfolgen.

Insbesondere kann die elektrische Schaltung so ausgestaltet sein, dass zur Ansteuerung einer ersten Bewegungsrichtung des Hubmagneten, insbesondere zum Ausfahren des Stifts, beide Energiespeicher in Serie über die zwei Spulen des Hubmagneten entladen werden, und zur Ansteuerung einer zweiten Bewegungsrichtung des Hubmagneten, insbesondere zum Einfahren des Stifts, der erste Energiespeicher in Serie über die zwei Spulen und der zweite Energiespeicher über nur eine der zwei Spulen, insbesondere über die erste Spule, entladen wird.

Weiter bevorzugt erfolgt die Entladung des zweiten Energiespeichers mit einer zeitlichen Verzögerung zur Entladung des ersten Energiespeichers erfolgt, wobei das Entladen des zweiten Energiespeichers bevorzugt noch vor Eintreten des Stellvorgangs einsetzt. Der Hubmagnet weist bevorzugt zwei Spulen auf, welche in Serie geschaltet sind und bevorzugt eine Mittelanzapfung aufweisen.

Weiterhin kann mindestens ein erster und einer zweiter Energiespeicher vorgesehen sein, wobei entlang einer ersten Bewegungsrichtung, insbesondere zum Ausfahren des Stifts, beide Energiespeicher über die in Reihe geschalteten Spulen entladen werden, während bei der umgekehrten Bewegungsrichtung, insbesondere zum Ausfahren des Stifts, zunächst der erste Energiespeicher über die in Reihe geschalteten Spulen entladen wird, und mit einer zeitlichen Verzögerung der zweite Energiespeicher über die Mittelanzapfung beider Spulen entladen wird, wobei das Entladen des zweiten Energiespeichers bevorzugt noch vor Eintreten des Stellvorgangs einsetzt.

In einer möglichen Ausführungsform weist die Steuerung Mittel zur Positionserfassung des Verriegelungselements auf. Insbesondere kann die Steuerung die mittels der Mittel zur Positionserfassung gewonnene Lageinformation beim Ansteuern des bistabilen Hubmagneten berücksichtigen. Bevorzugt weist sie hierfür einen Mikro-Controller auf, welcher mit den Mitteln zur Positionserfassung verbunden ist.

In einer möglichen Ausführungsform weist die Steuerung Mittel zur Erfassung der Winkelstellung des Gelenkes auf, wobei die Steuerung das Verriegelungselement anhand der durch die Mittel zur Erfassung der Winkelstellung gewonnenen Daten ansteuert. Bevorzugt weist sie hierfür einen Mikro-Controller auf, welcher mit den Mitteln zur Erfassung der Winkelstellung verbunden ist. Bevorzugt schaltet die Steuerung die Versorgungsspannung für das Verriegelungselement ein und aus. Dieser Teil der Steuerung kann auch in einer übergeordneten Steuerung der Gelenk-Orthese oder Prothese angeordnet sein, welche die Steuerung des Verriegelungselementes ansteuert, insbesondere durch Ein- und Ausschalten der Versorgungsspannung.

In einer bevorzugten Ausführungsform werden die Energiespeicher der Steuerung über eine Batterie und/oder einen Akkumulator geladen. Bevorzugt ist die Batterie und/oder der Akkumulator an der Gelenk-Orthese oder Gelenk-Prothese angeordnet und stellt die Versorgungsspannung für das Verriegelungselement zur Verfügung.

Weiterhin kann der Stift einen Dämpfungsmechanismus aufweisen und/oder mit einem solchen verbunden sein. Der Dämpfungsmechanismus bringt das Gelenk bevorzugt gedämpft in eine verriegelte Position.

In einer möglichen Ausführungsform weist das Verriegelungselement ein Federsystem mit einer ersten Feder, welche in einer ersten Hubendlage auf den oder die Anker des Hubmagneten eine Kraft in Richtung die Hubmittellage ausübt, sowie mit einer zweiten Feder, welche in einer zweiten Hubendlage auf den oder die Anker eine Kraft in Richtung die Hubmittellage ausübt, auf, wobei der oder die Anker im stromlosen Fall in beiden Hubendlagen entgegen der Federkraft permanentmagnetisch gehalten werden. Das Federsystem kann in den Hubmagneten integriert sein oder einen separaten Teil des Verriegelungselements bilden. Bevorzugt weisen die erste und die zweite Feder unterschiedlich lange Federwege auf und/oder üben in der jeweiligen Hubendlage unterschiedlich große Kräfte auf den oder die Anker aus und/oder weisen unterschiedlich große Federraten auf.

Wie oben beschrieben entspricht die zweite Hubendlage bevorzugt der komplett ausgefahrenen Position des Stifts und die erste Hubendlage der komplett eingefahrenen Position.

Bevorzugt ist der Federweg der ersten Feder größer als der Federweg der zweiten Feder und die zweite Feder übt in der zweiten Hubendlage eine größere Kraft auf den oder die Anker aus als die erste Feder in der ersten Hubendlage auf den oder die Anker ausübt.

Alternativ oder zusätzlich kann der Federweg der ersten Feder größer als der Federweg der zweiten Feder sein und die Federrate der zweiten Feder in der zweiten Hubendlage größer als die Federrate der ersten Feder in der ersten Hubendlage sein.

In einer möglichen Ausführungsform der vorliegenden Erfindung beträgt der Federweg der ersten Feder zwischen dem 2-fachen und dem 100-fachen des Federweges der zweiten Feder, bevorzugt zwischen dem 4-fachen und dem 20-fachen.

In einer weiteren möglichen Ausführungsform der vorliegenden Erfindung beträgt die Kraft, welche die zweite Feder in der zweiten Hubendlage auf den oder die Anker ausübt, zwischen dem 1 ,5-fachen und dem 100-fachen der Kraft, welche die erste Feder in der ersten Hubendlage auf den oder die Anker ausübt, bevorzugt zwischen dem 3-fachen und dem 15-fachen.

In einer weiteren möglichen Ausführungsform der vorliegenden Erfindung beträgt die Federrate der zweiten Feder in der zweiten Hubendlage zwischen dem 2-fachen und dem 1000-fachen der Federrate der ersten Feder in der ersten Hubendlage, bevorzugt zwischen dem 10-fachen und dem 500-fachen, weiter bevorzugt zwischen dem 20-fachen und dem 100-fachen.

In einer weiteren möglichen Ausführungsform erzeugt mindestens eine der Federn und bevorzugt die zweite Feder über einen Teil des Hubweges keine Kraft zwischen dem Anker und dem Stator und/oder steht über einen Teil des Hubweges nicht mit dem Anker und/oder dem Stator in Kontakt. Bevorzugt ist in diesem Fall eine Rückhaltesicherung vorgesehen, welche die Feder über diesen Teil des Hubweges in einer vorgegebenen Position sichert und dabei bevorzugt in vorgespanntem Zustand hält.

In einer möglichen Ausführungsform ist vorgesehen, dass die magnetische Haltekraft des Hubmagneten in einer der beiden Hubendlagen kleiner als in der anderen Hubendlage ist. Insbesondere kann die magnetische Haltekraft des Hubmagneten in einer der beiden Hubendlagen um mindestens 20% kleiner, weiter bevorzugt um mindestens 30% kleiner als in der anderen Hubendlage sein. Bevorzugt ist die magnetische Haltekraft in der ersten Hubendlage kleiner als in der zweiten Hubendlage.

Alternativ oder zusätzlich kann die magnetische Haltekraft in einer der Hubendlagen mindestens 20% der magnetischen Haltekraft in der anderen Hubendlage betragen, bevorzugt mindestens 30%.

In einer möglichen Ausführungsform ist vorgesehen, dass der Stator und der oder die Anker in einer der Hubendlagen und bevorzugt in der ersten Hubendlage eine geometrische Kennlinienbeeinflussung aufweisen, insbesondere einen nicht in einer Ebene senkrecht zur Achse des Hubmagneten verlaufenden Arbeits-Luftspalt, insbesondere einen konisch verlaufenden Arbeits-Luftspalt.

Bevorzugt weisen der Stator und der oder die Anker in der anderen Hubendlage und bevorzugt in der zweiten Hubendlage eine schwächere oder keine geometrische Kennlinienbeeinflussung auf.

In einer möglichen Ausführungsform ist vorgesehen, dass sich die Differenz zwischen dem Betrag der magnetischen Haltekraft und dem Betrag der Kraft, welche die jeweilige Feder aufbringt, in den beiden Hubendlagen um maximal 50% des größeren Wertes unterscheidet.

Im Folgenden werden einige konstruktive Merkmale eines Hubmagneten beschrieben, wie er in dem erfindungsgemäßen Verriegelungselement eingesetzt werden kann. Die Merkmale können sowohl einzeln, als auch in Kombination verwirklicht sein:
In einer möglichen Ausführungsform ist vorgesehen, dass die mindestens eine Spule und der mindestens eine Permamentmagent am Stator angeordnet sind. In einer möglichen Ausführungsform ist vorgesehen, dass der Stator ein Gehäuse bildet, welches den oder die Anker umgibt, wobei bevorzugt ein Anker vorgesehen ist, welcher im Inneren des Stators auf einer Führungsstange angeordnet ist, wobei die Führungsstange bevorzugt beweglich am Stator gelagert ist. Die Führungsstange steht bevorzugt mit dem Stift in Verbindung und überträgt die Kraft des Hubmagenten auf den Stift.

In einer möglichen Ausführungsform ist vorgesehen, dass das Federsystem innerhalb des Stators angeordnet ist, wobei die erste Feder bevorzugt zwischen dem ersten Stirnabschnitt und einer ersten Seite des Ankers und die zweite Feder zwischen einem zweiten Stirnabschnitt und einer zweiten Seite des Ankers angeordnet ist, und/oder wobei die erste und die zweite Feder als Spiraldruckfedern ausgeführt sind, welche die Führungsstange des Ankers umfassen.

In einer möglichen Ausführungsform ist vorgesehen, dass der Stator eine weichmagnetische Hülse und einen ersten und zweiten weichmagnetischen Stirnabschnitt aufweist, welche ein Gehäuse bilden, in welchem der Anker verschieblich angeordnet ist.

In einer möglichen Ausführungsform kann zwischen dem Anker und dem ersten Stirnabschnitt mindestens ein erster Arbeitsluftspalt und zwischen dem Anker und dem zweiten Stirnabschnitt mindestens ein zweiter Arbeitsluftspalt vorgesehen sein.

Bevorzugt sind am Stator mindestens ein Permanentmagnet und mindestens eine erste und eine zweite Spule angeordnet sind, wobei der Anker in der ersten Hubendlage mit der Hülse und dem ersten Stirnabschnitt einen ersten magnetischen Teilkreis bildet, welcher zumindest die erste Spule umgibt, während der oder die Arbeitsluftspalte mit dem zweiten Stirnabschnitt maximal geöffnet sind, und wobei der Anker in der zweiten Hubendlage mit der Hülse und dem zweiten Stirnabschnitt einen zweiten magnetischen Teilkreis bildet, welcher zumindest die zweite Spule umgibt, während der oder die Arbeitsluftspalte mit dem ersten Stirnabschnitt maximal geöffnet sind.

In einer möglichen Ausführungsform ist vorgesehen, dass der mindestens eine Permanentmagnet in axialer Richtung zwischen der ersten und der zweiten Spule angeordnet ist und jeweils einen Teil des ersten und des zweiten magnetischen Teilkreises bildet, wobei der Permanentmagnet so angeordnet ist, dass er sowohl in der ersten als auch in der zweiten Hubendlage in axialer Richtung mit dem Anker überlappt und diesen bevorzugt umgibt, wobei der Permanentmagnet bevorzugt magnetisch unmittelbar an den Anker koppelt. Es sind jedoch andere Anordnungen des Permanentmagneten oder der Permanentmagneten möglich.

In einer bevorzugten Ausgestaltung ist dagegen vorgesehen, dass mindestens ein erster und ein zweiter Permanentmagnet vorgesehen sind, wobei die erste und die zweite Spule in axialer Richtung zwischen dem ersten und dem zweiten Permanentmagneten angeordnet sind, wobei der erste Permanentmagnet die Hülse und den ersten Stirnabschnitt und der zweite Permanentmagnet die Hülse und den zweiten Stirnabschnitt unter eine magnetische Spannung setzen. Hierdurch kann die Baulänge gegenüber anderen konstruktiven Ausgestaltungen verringert werden.

In einer möglichen Ausführungsform ist vorgesehen, dass der erste Teilmagnetkreis den ersten Permanentmagneten und der zweite Teilmagnetkreis den zweiten Permanentmagneten umfasst.

In einer möglichen Ausführungsform ist vorgesehen, dass der Anker die Hülse und den ersten Stirnabschnitt in der ersten Hubendlage magnetisch kurzschließt und der Anker die Hülse und den zweiten Stirnabschnitt in der zweiten Hubendlage magnetisch kurzschließt.

In einer möglichen Ausführungsform ist vorgesehen, dass die Hülse zwischen den beiden Spulen einen Magnetkreisabschnitt aufweist, welcher sowohl in der ersten als auch in der zweiten Hubendlage in axialer Richtung mit dem Anker überlappt und diesen bevorzugt umgibt, wobei der Magnetkreisabschnitt bevorzugt magnetisch unmittelbar an den Anker koppelt.

In einer möglichen Ausführungsform ist vorgesehen, dass die erste und die zweite Spule zumindest teilweise zwischen der Hülse und dem Bewegungsbereich des Ankers und/oder in einer Innennut und/oder Aussparung der Hülse angeordnet sind.

Weiterhin bevorzugt ist vorgesehen, dass der erste und/oder zweite Stirnabschnitt einen Befestigungsbereich aufweisen, welcher sich in radialer Richtung über den ersten bzw. zweiten Permanentmagneten hinweg erstreckt und an der Hülse befestigt ist. Hierdurch wird die Montage erheblich vereinfacht. Bevorzugt ist der Befestigungsbereich durch den ersten bzw. zweiten Permanentmagneten magnetisch gesättigt.

In einer möglichen Ausführungsform ist der Befestigungsbereich plattenförmig, insbesondere ringplattenförmig ausgestaltet, und/oder weist Aussparungen auf. In einer möglichen Ausführungsform weist der Befestigungsbereich nach außen hin weniger Material aufweist und wird insbesondere dünner.

Die vorliegende Erfindung umfasst in einem zweiten, unabhängigen Aspekt eine Steuerung für ein Verriegelungselement, wie sie oben im Kontext des Verriegelungselements beschrieben wurde. Insbesondere weist die Steuerung Schalter auf, über welche Energiespeicher über die mindestens eine Spule des Hubmagneten so entladen werden können, dass der Stift ausgefahren wird. Bevorzugt ist die Steuerung dabei so ausgeführt, wie dies oben bereits beschrieben wurde.

Insbesondere weist die Steuerung in einer möglichen Ausführungsform mindestens einen ersten und einen zweiten elektrischen Energiespeicher auf, wobei der erste Energiespeicher in Serie über zwei Spulen des Hubmagneten entladbar ist, und wobei der zweite Energiespeicher über nur eine der zwei Spulen des Hubmagneten entladbar ist. In einer möglichen Ausführungsform ist der zweite Energiespeicher wahlweise über eine der beiden Spulen entladbar. Insbesondere kann die Entladung dabei je nach Bewegungsrichtung über die erste oder die zweite Spule erfolgen.

Alternativ oder zusätzlich kann der zweite Energiespeicher wahlweise auch in Serie über die zwei Spulen des Hubmagneten entladbar sein. Insbesondere kann die Entladung dabei je nach Bewegungsrichtung über eine der beiden Spulen oder in Serie über die zwei Spulen erfolgen.

Insbesondere kann die elektrische Schaltung so ausgestaltet sein, dass zur Ansteuerung einer ersten Bewegungsrichtung des Hubmagneten, insbesondere zum Ausfahren des Stifts, beide Energiespeicher in Serie über zwei Spulen des Hubmagneten entladen werden, und zur Ansteuerung einer zweiten Bewegungsrichtung des Hubmagneten, insbesondere zum Einfahren des Stifts, der erste Energiespeicher in Serie über die zwei Spulen und der zweite Energiespeicher über nur eine der zwei Spulen, insbesondere über die erste Spule, entladen wird.

Weiter bevorzugt erfolgt die Entladung des zweiten Energiespeichers mit einer zeitlichen Verzögerung zur Entladung des ersten Energiespeichers erfolgt, wobei das Entladen des zweiten Energiespeichers bevorzugt noch vor Eintreten des Stellvorgangs einsetzt.

Die vorliegende Erfindung umfasst weiterhin Gelenk-Orthese oder Gelenk-Prothese, mit einem ersten Element und einem zweiten Element, welche über ein Drehgelenk schwenkbar miteinander verbunden sind, und mit einem elektromagnetischen Verriegelungselement, wie es oben beschrieben wurde, zum Verriegeln des Drehgelenkes.

Insbesondere kann das Verriegelungselement zum Verriegeln des Drehgelenkes in einer gestreckten Position der Gelenk-Orthese bzw. Gelenk-Prothese eingesetzt werden. Bevorzugt ist der Hubmagnet dabei am ersten oder zweiten Element angeordnet, insbesondere im Bereich des Drehgelenks.

Weiterhin kann der Stift mit einem Verriegelungsgegenstück zusammenwirken, um das Gelenk zu verriegeln. Bevorzugt ist das Verriegelungsgegenstück an dem anderen Element angeordnet, insbesondere im Bereich des Drehgelenks.

In einer möglichen Ausführungsform weist die Gelenk-Orthese oder Gelenk-Prothese Mittel zur Erfassung der Winkelstellung des Dreh-Gelenkes auf, wobei die Steuerung das Verriegelungselement anhand der durch die Mittel zur Erfassung der Winkelstellung gewonnenen Daten ansteuert. Bevorzugt weist sie hierfür einen Mikrocontroller auf, welcher mit den Mitteln zur Erfassung der Winkelstellung verbunden ist. Bevorzugt schaltet die Steuerung die Versorgungsspannung für das Verriegelungselement ein und aus. Die Steuerung kann eine übergeordnete Steuerung der Gelenk-Orthese oder Prothese darstellen, welche die Steuerung des Verriegelungselementes ansteuert, insbesondere durch Ein- und Ausschalten der Versorgungsspannung.

In einer bevorzugten Ausführungsform werden die Energiespeicher des Verriegelungselementes über eine Batterie und/oder einen Akkumulator geladen. Bevorzugt ist die Batterie und/oder der Akkumulator an der Gelenk-Orthese oder Gelenk-Prothese angeordnet, insbesondere am gleichen Element wie das Verriegelungselement, und stellt die Versorgungsspannung für das Verriegelungselement zur Verfügung.

Insbesondere handelt es sich bei der Gelenk-Orthese bzw. Gelenk-Prothese um eine Knie-Orthese oder Knie-Prothese. Das erste Element ist in diesem Fall am Oberschenkel anordenbar, das zweite Element stützt oder ersetzt den Unterschenkel. Die vorliegende Erfindung wird nun anhand von Ausführungsbeispielen und Zeichnungen näher erläutert.

Dabei zeigen:
Fig. 1 : ein Ausführungsbeispiel einer Gelenk-Orthese oder Prothese gemäß der vorliegenden Erfindung,
Fig. 2: ein Ausführungsbeispiel des erfindungsgemäß eingesetzten Hubmagneten in einer Schnittansicht,
Fig. 3: ein erstes Ausführungsbeispiel einer Steuerung zur Ansteuerung des erfindungsgemäß eingesetzten bistabilen Hubmagneten und
Fig. 4: ein zweites Ausführungsbeispiel einer Steuerung zur Ansteuerung des erfindungsgemäß eingesetzten bistabilen Hubmagneten.

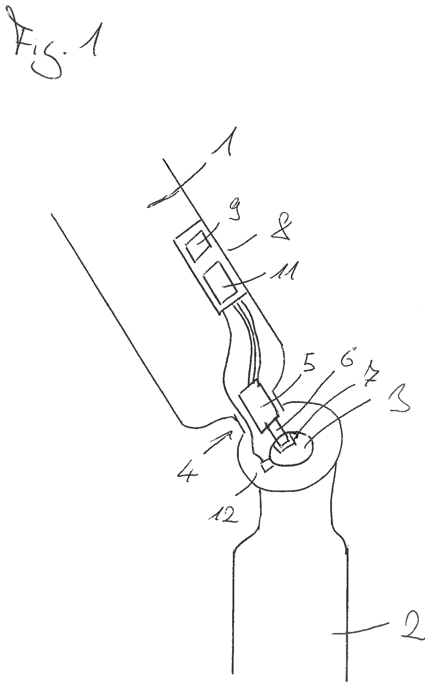

In Fig. 1 ist schematisch ein Ausführungsbeispiel einer erfindungsgemäßen Gelenk-Orthese oder Prothese gezeigt. Die Gelenk-Orthese oder Gelenk-Prothesen weist ein erstes Element 1 und ein zweites Element 2 auf, welche über ein Drehgelenk 3 schwenkbar miteinander verbunden sind. Insbesondere handelt es sich dabei um eine Knie-Orthese oder eine Knie-Prothese. Das erste Element 1 ist in diesem Fall am Oberschenkel anordenbar, das zweite Element 2 stützt oder ersetzt den Unterschenkel.

Das erfindungsgemäße Verriegelungselement 4 wird zum Verriegeln des Drehgelenkes 3 eingesetzt. Hierfür weist es einen Stift 6 auf, welcher ein- und ausgefahren werden kann, und in seiner ausgefahrenen Position das Drehgelenk verriegelt und in seiner eingefahrenen Position das Drehgelenk freigibt. Hierfür ist ein Verriegelungsgegenelement 7 vorgesehen, mit welchem der Stift 6 zusammenwirkt, um das Drehgelenk zu verriegeln. Ein Einfahren des Verriegelungselementes im Sinne der vorliegenden Erfindung erfordert nicht, dass der Stift in eine Aussparung eines Gehäuses eingefahren wird. Vielmehr liegt ein Einfahren bereits dann vor, wenn der Stift aus der ausgefahrenen Position zurück in eine Position gefahren wird, in welcher er das Drehgelenk frei gibt.

Solche Gelenk-Orthesen werden für Patienten eingesetzt, welche das Kniegelenk nicht oder nicht mehr ausreichend kontrollieren können. Durch die Verriegelbarkeit des Drehgelenkes wird es solchen Patienten ermöglicht, dennoch zu laufen oder zu stehen.

Insbesondere kann das Drehgelenk in einer gestreckten Position der Gelenk-Orthese verriegelt werden, so dass die Orthese ein sicheres Stehen oder eine sichere Stütze beim Laufen bietet. In gleicher Art kann eine Gelenk-Prothese einen fehlenden Unterschenkel ersetzen. Hierfür sind das Verriegelungselement und das Verriegelungsgegenstück in einer entsprechenden Position relativ zueinander an dem ersten und dem zweiten Element angeordnet.

In einer möglichen Ausführungsform können auch mehrere Verriegelungsgegenstücke vorgesehen sein, so dass das Gelenk in unterschiedlichen Positionen verriegelt werden kann.

Im Ausführungsbeispiel ist das Verriegelungselement 4 am ersten Element 1 angeordnet, das Verriegelungsgegenstück am zweiten Element 2.

Der Stift 6 ist an dem Gehäuse 5 des elektromagnetischen Verriegelungselements beweglich angeordnet und kann erfindungsgemäß mit Hilfe eines bistabilen Hubmagneten ein- und ausgefahren werden. Das Gehäuse ist an dem ersten Element befestigt. Eine Achse 50 des Hubmagneten 10 steht mit dem Stift 6 in Verbindung, um diesen ein- und auszufahren. Alternativ kann der Stift durch eine Achse 50 des Hubmagneten gebildet werden.

Weiterhin ist eine Steuerung 8 zu Ansteuerung des Hubmagneten 10 vorgesehen. Diese weist einen oder mehrere elektrische Energiespeicher 9, im Ausführungsbeispiel Kondensatoren, auf. Weiterhin sind Schalter 11, im Ausführungsbeispiel Halbleiterschalter, vorgesehen, welche den oder die Energiespeicher 9 über mindestens eine Spule des Hubmagneten so entladen können, dass der Stift ausgefahren wird.

Die Steuerung 8 erkennt bevorzugt eine Unterbrechung und/oder ein Abschalten der Spannungsversorgung des Verriegelungselements und fährt in Reaktion hierauf den Stift aus, wobei bevorzugt das Abfallen der Versorgungsspannung mittels einer Flankenerkennung erkannt wird. Weiterhin kann die Steuerung 8 so ausgestaltet sein, dass auf ein Zuschalten der Versorgungsspannung hin der oder die elektrischen Energiespeicher aufgeladen werden, und dass das Erreichen einer bestimmten Schwellspannung am elektrischen Energiespeicher von der Steuerung erkannt wird, woraufhin selbige den oder die Energiespeicher über den bistabilen Hubmagneten so entlädt, dass der Stift eingefahren wird.

Der Hubmagnet weist neben der mindestens einen Spule mindestens einen Permanentmagneten auf, und ist bistabil ausgestaltet. Insbesondere wird der Hubmagnet sowohl in einer ersten Hubendlage, welche einem komplett eingefahrenen Stift entspricht, als auch in einer zweiten Hubendlage, in welcher der Stift komplett ausgefahrenen ist, permanentmagnetisch gehalten, solang die Spulen des Hubmagneten unbestromt bleiben. Bevorzugt weist der Hubmagnet ein Federsystem auf, welches den Hubmagneten in den Hubendlagen in Richtung auf eine Hubmittellage vorspannt.

Die Gelenk-Orthese bzw. Gelenk-Prothese weist weiterhin eine übergeordnete Steuerung auf, welche das Verriegelungselement ansteuert, bevorzugt durch Ein- und Ausschalten der Versorgungsspannung. Eine unmittelbare Ansteuerung der Schalter der Steuerung des Verriegelungselementes ist jedoch ebenfalls denkbar. Die Versorgungsspannung zum Laden der Energiespeicher, insbesondere der Kondensatoren, wird bevorzugt über Batterien und/oder Akkumulatoren zur Verfügung gestellt. Bevorzugt sind diese und die Steuerung der Gelenk-Orthese bzw. Gelenk-Prothese am ersten Element angeordnet. Im Ausführungsbeispiel ist weiterhin ein Sensor 12 vorgesehen, welcher die Winkel-Position des Drehgelenks erfasst. Die Daten des Sensors 12 können über die Steuerung der Gelenk-Orthese bzw. Gelenk-Prothese ausgewertet werden und zur Ansteuerung des Verriegelungselementes herangezogen werden.

Im Ausführungsbeispiel weist das Verriegelungselement im unbestromten Fall eine Raststelle in einer Position auf, in welcher der Stift teilweise ausgefahren ist. Insbesondere wird dies über eine weitere stabile Hublage des Hubmagneten erreicht, welche zwischen der ersten und der zweiten Hubendlage liegt. Bevorzugt ist der Stift an der Raststelle hinreichend weit ausgefahren, um das Drehgelenk zu verriegeln. Insbesondere kann der Stift über seine Mittellage hinaus ausgefahren sein, und bevorzugt auf mindestens 70% seines Hubwegs ausgefahren sein.

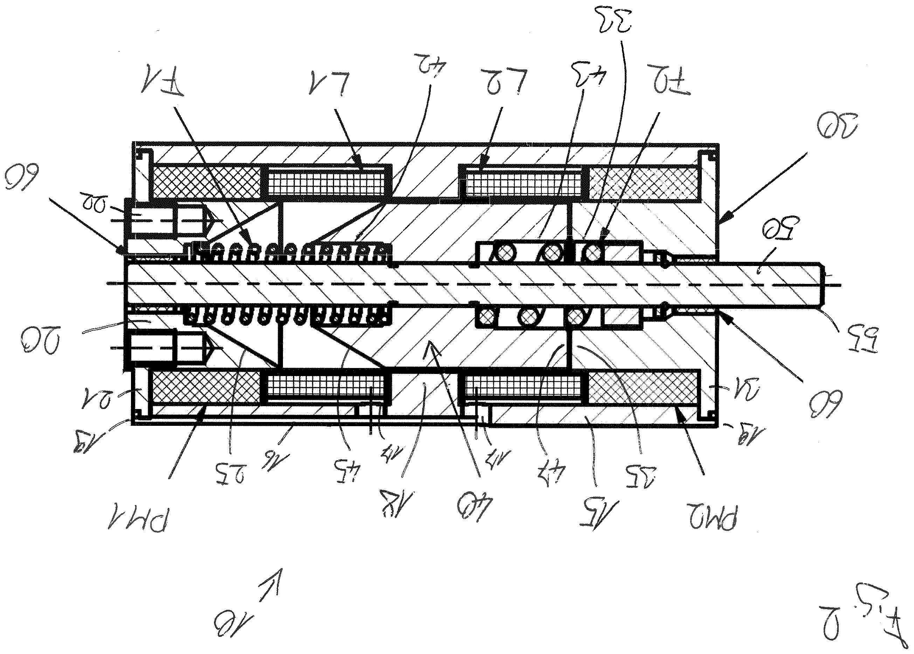

Ein Ausführungsbeispiel eines bistabilen Hubmagneten, wie er im Rahmen der vorliegenden Erfindung zum Einsatz kommen kann, sowie zwei Ausführungsbeispiele von Steuerungen zur Ansteuerung eines erfindungsgemäß eingesetzten Hubmagneten, werden im Folgenden anhand der Fig. 2 bis 4 näher dargestellt.

Fig. 2 zeigt ein Ausführungsbeispiel eines bistabilen Hubmagneten, bei welchen eine Mehrzahl von Aspekten der vorliegenden Erfindung in Kombination verwirklicht sind. Die anhand des Ausführungsbeispiels in Kombination beschriebenen Merkmale gemäß der einzelnen Aspekte können jedoch auch jeweils für sich genommen erfindungsgemäß eingesetzt werden.

Der bistabile Hubmagnet gemäß der vorliegenden Erfindung weist einen Stator und einen gegenüber dem Stator axial verschieblichen Anker 40 auf. Stator und Anker bestehen aus einem weichmagnetischen Material.

Im Ausführungsbeispiel umfasst der Stator eine weichmagnetische Hülse 15 und zwei weichmagnetische Stirnabschnitte 20 und 30, welche ein Gehäuse bilden, in welchem der Anker 40 verschieblich angeordnet ist. Die Stirnabschnitte weisen im Ausführungsbeispiel jeweils einen Bereich auf, welcher in der Hülse 15 angeordnet ist, insbesondere einen im wesentlichen zylinderförmigen Bereich.

Im Ausführungsbeispiel wird der Anker 40 von einer Achse 50 getragen, welche über Lager 60 an den Stirnabschnitten 20 und 30 des Stators axialverschieblich gelagert ist. Durch eine Bewegung des Ankers 40 wird dementsprechend die Achse 50 bewegt. Im Ausführungsbeispiel weist die Achse 50 eine zweite Seite mit einen Verbindungsbereich 55 auf, mit welchem sie mit dem Stift verbunden wird. Alternativ kann die Achse 50 unmittelbar als Stift dienen, wobei der Bereich 55 bevorzugt mit einem Verriegelungsgegenelement zusammen wirkt. Zwischen dem Anker 40 und den Stirnabschnitten 20 und 30 befinden sich die Arbeitsluftspalte des Hubmagneten.

In der zweiten Hubendlage ist die zweite Seite der Achse 50 mit dem Verbindungsbereich 55 ganz ausgefahren, so dass auch der Stift ganz ausgefahren ist. In der ersten Hubendlage ist die zweite Seite der Achse 50 mit dem Verbindungsbereich 55 zum Stift komplett eingefahren und dafür die Achse auf der gegenüberliegenden ersten Seite komplett ausgefahren ist. In der ersten Hubendlage ist der Stift komplett eingefahren.

Der Hubmagnet weist im Ausführungsbeispiel Bohrungen 22 auf, insbesondere Gewindebohrungen, durch welche er an der Gelenk-Orthese bzw. Prothese montiert ist.

Alternative konstruktive Ausgestaltungen des Stators, des Ankers sowie der Achse sind im Rahmen der vorliegenden Erfindung ebenfalls denkbar.

Der innere Aufbau des Hubmagneten ist in der Schnittansicht in Fig. 2 gezeigt. Der bistabile Hubmagnet weist ein Federsystem mit einer ersten Feder F1 auf, welche in einer ersten Hubendlage auf den Anker 40 eine Kraft in Richtung auf die Hubmittellage ausübt, sowie eine zweite Feder F2, welche in der in Fig. 2 dargestellten zweiten Hubendlage eine Kraft auf den Anker 40 in Richtung auf die Hubmittellage ausübt.

Im Ausführungsbeispiel sind die beiden Federn jeweils innerhalb des durch den Stator gebildeten Gehäuses zwischen einem der Stirnabschnitte 20 bzw. 30 und dem Anker 40 angeordnet. Im Ausführungsbeispiel handelt es sich um Spiralfedern, welche die Achse 50 umgeben. Im Anker 40 sind Ringnuten 42 bzw. 43 vorgesehen, welche in den jeweiligen Endlagen zumindest einen Teil der jeweiligen Feder aufnehmen. Entsprechende Ringnuten können auch in den Stirnabschnitten 20 und 30 vorgesehen sein.

Weiterhin ist mindestens ein Permanentmagnet PM1 und PM2 vorgesehen, welcher im unbestromten Zustand der Spulen den Anker 40 entgegen der Kraft der jeweiligen Feder in der jeweiligen Hubendlage hält. Im Ausführungsbeispiel sind zwei Permanentmagnete PM1 und PM2 vorgesehen, welche den jeweiligen Hubendlagen zugeordnet sind. Anstelle zweier Permanentmagnete könnte auch nur ein einzelner Permanentmagnet eingesetzt werden.

Weiterhin sind Spulen L1 und L2 vorgesehen, durch deren Bestromung der Anker von einer Hubendlage in die andere Hubendlage verfahren werden kann. Im Ausführungsbeispiel sind zwei Spulen L1 und L2 vorgesehen, deren Wicklungen im Bereich 17 jeweils separat aus dem Gehäuse geführt sind. Alternativ könnten die Spulen auch innerhalb des Gehäuses in Reihe geschaltet sein und bevorzugt einen Mittelabgriff aufweisen.

Gemäß dem ersten Aspekt der vorliegenden Erfindung werden unterschiedliche Federn F1 und F2 eingesetzt. Im Ausführungsbeispiel weisen die erste und die zweite Feder zum einen unterschiedlich lange Federwege auf. Insbesondere ist der Federweg der ersten Feder F1 größer als der Federweg der zweiten Feder F2. Weiterhin üben die beiden Federn in der jeweiligen Hubendlage unterschiedlich große Kräfte auf den Anker aus. Insbesondere übt die erste Feder F1 in der ersten Hubendlage, in welche der Anker 40 in Anschlag mit dem ersten Stirnabschnitt 20 steht, eine kleinere Kraft auf den Anker 40 aus, als dies die zweite Feder F2 in der zweiten, in Fig. 2 gezeigten Hubendlage tut, in welcher der Anker 40 in Anschlag mit dem zweiten Stirnabschnitt 30 steht. Weiterhin weist die erste Feder F1 im Ausführungsbeispiel eine kleinere Federrate auf als die zweite Feder F2.

Weiterhin übt die zweite Feder aufgrund des kleineren Federwegs nur über einen Teil des Hubweges eine Kraft auf den Anker 40 aus. Bevorzugt ist eine in Fig. 2 nicht eingezeichnete Rückhaltesicherung vorgesehen, welche die zweite Feder F2 über den Teil des Hubweges, in welchem diese keine Kraft zwischen dem Anker und dem Stator erzeugt, in einer vorgegebenen Position sichert und diese in einem vorgespannten Zustand hält. Dies erhöht die Lebensdauer des Hubmagneten.

Im konkreten Ausführungsbeispiel weist der Hubmagnet einen Hubweg von 15mm auf. Die erste Feder weist einen Federweg auf, welche dem Hubweg entspricht. Die zweite Feder F2 weist dagegen lediglich einen Federweg von 2mm auf. Die erste Feder übt in der ersten Hubendlage eine Kraft von ca. 5 N auf den Anker auf und weist eine Federrate von ca. 0,35 N/mm auf. Die zweite Feder übt in der zweiten Hubendlage eine Kraft von ca. 35 N auf den Anker auf und weist eine Federrate von ca. 17 N/mm auf. Beide Federn sind bei Erreichen ihres maximalen Federwegs vorgespannt.

Durch die unterschiedlichen Federn F1 und F2 können im Ausführungsbeispiel eine Reihe von Vorteilen erzieht werden. Die starke Feder F2 sorgt für eine hohe Beschleunigung des Ankers bei einer Bewegung aus der zweiten Hubendlage in Richtung auf die Hubmittellage. Die erste Feder F1 mit dem langen Federweg erlaubt dagegen eine entsprechend lange Ausgestaltung des Hubweges.

Gemäß einem weiteren Aspekt der vorliegenden Erfindung weist der Hubmagnet im unbestromten Fall eine asymmetrisch angeordnete Raststelle auf. Diese Raststelle stellt eine dritte stabile Hublage des bistabilen Hubmagneten im unbestromten Fall dar, welche zwischen der ersten und der zweiten Hubendlage angeordnet ist. Diese Raststelle, in welcher sich die von den Federn und Permanentmagneten auf den Anker 40 ausgeübten entgegengesetzten Kräfte aufheben, ist asymmetrisch, d.h. gegenüber der Mitte des Hubweges versetzt angeordnet.

Dies hat den Vorteil, dass der Hubmagnet mit nur sehr wenig Energie in eine weitgehend aus- oder eingefahrene Stellung gebracht werden kann, indem er von der Hubendlage, welche weiter von der Raststelle entfernt ist, in die Raststelle verfahren wird. Eine solche asymmetrische Raststelle, welche mit nur geringem Energieeinsatz angefahren werden kann, stellt in vielen Anwendungen eine wichtige Sicherungsfunktion dar.

Im Ausführungsbeispiel wird die asymmetrische Raststelle hauptsächlich durch die unterschiedlichen Federn gemäß dem ersten Aspekt der vorliegenden Erfindung erreicht, insbesondere durch die unterschiedlich langen Federwege und/oder die unterschiedlich großen Kräfte und/oder unterschiedlich großen Federraten der ersten und der zweiten Feder. Insbesondere ist die Raststelle näher an der zweiten Hubendlage angeordnet als an der ersten Hubendlage, da die zweite Feder einen kleineren Federweg aufweist als die erste Feder. Da die zweite Feder eine weitaus größere Federrate aufweist als die erste Feder, wird die Raststelle überwiegend durch die Länge des Federwegs der zweiten Feder bestimmt, und liegt daher im Ausführungsbeispiel ca. 2mm von der zweiten Hubendlage entfernt. Im Ausführungsbeispiel üben die auf den Anker wirkenden magnetischen Kräfte nur eine untergeordnete Rolle auf die genaue Lage der Raststelle aus.

Die Raststelle kann aus der ersten Hubendlage mit nur geringem Energieeinsatz erreicht werden, da hierzu die (große) rückstellende Kraft der zweiten Feder F2 nicht überwunden werden muss. Dennoch ist der Antrieb bei Erreichen der Raststelle bereits weitgehend ausgefahren.

Gemäß einem weiteren Aspekt der vorliegenden Erfindung ist der Hubmagnet so ausgestaltet, dass die permanentmagnetische Haltekraft, oft auch als "Haftkraft" bezeichnet, in der ersten und der zweiten Hubendlage unterschiedlich groß ist. Insbesondere ist der Hubmagnet dabei so ausgestaltet, dass die permanentmagnetische Haltekraft in der ersten Hubendlage kleiner ist als in der zweiten Hubendlage. Im Ausführungsbeispiel ist hierfür eine geometrische Kennlinienbeeinflussung zwischen der ersten Stirnseite 45 des Ankers, welche dem ersten Stirnabschnitt 20 zugewandt ist, und der Innenseite 25 des ersten Stirnabschnittes 20 vorgesehen. Zwischen diesen beiden Flächen 25 und 45 befindet sich der erste Arbeitsluftspalt, welcher in der ersten Hubendlage geschlossen ist. Die geometrische Kennlinienbeeinflussung bedeutet, dass die Flächen 25 und 45 nicht in einer Ebene senkrecht zur axialen Bewegungsrichtung des Hubmagneten verlaufen, sondern bezüglich einer solchen Ebene ein Profil aufweisen. Im Ausführungsbeispiel weisen die Flächen ein konisches Profil auf, welches im Ausführungsbeispiel einen solchen Winkel aufweist, dass die permanentmagnetische Haltekraft um näherungsweise 50% reduziert wird.

Auf der gegenüberliegenden Seite, auf welcher sich die zweite Stirnseite 47 des Ankers 40 und die Innenseite 35 des zweiten Stirnabschnittes 30 über einen zweiten Arbeitsluftspalt gegenüber liegen, ist dagegen keine geometrische Kennlinienbeeinflussung vorgesehen. Hier verlaufen die beiden Flächen, zwischen welchen sich der Arbeitsluftspalt befindet, in einer Ebene senkrecht zur axialen Bewegungsrichtung des Hubmagneten.

Die unterschiedlich großen permanentmagnetischen Haltekräfte in der ersten und in der zweiten Hubendlage sind bevorzugt so gewählt, dass die jeweilige Differenz zwischen der permanentmagnetischen Haltekraft und der jeweiligen entgegengesetzten Federkraft in den beiden Hubendlagen im Wesentlich gleich groß ist und/oder unter Berücksichtigung äußerer auf den Hubmagneten wirkender Kräfte bevorzugt zumindest in der gleichen Größenordnung liegt. Diese Differenz sichert den Hubmagneten in den beiden Hubendlagen jeweils gegen eine ungewollte Auslösung, bspw. durch Erschütterungen. Im Ausführungsbeispiel beträgt die magnetische Haltekraft in der ersten Hubendlage ca. 25 N, in der zweiten Hubendlage ca. 50 N. Gemäß einem weiteren Aspekt der vorliegenden Erfindung ist der bistabile Hubmagnet so ausgelegt, dass der Wert der in den beiden Hubendlagen jeweils im Hubmagneten gespeicherten potentiellen Energie sich um nicht mehr als 50% des größeren Wertes voneinander unterscheidet, d.h. dass der kleinere der beiden Werte mindestens 50% des größeren Wertes beträgt. Bevorzugt ist die potentielle Energie in den beiden Hubendlagen dabei im Wesentlichen gleich groß. Für die Berechnung der potentiellen Energie wird die elektrische Energie unberücksichtigt gelassen, und der unbestromte Fall betrachtet. Im einfachsten Fall ergibt sich die potentielle Energie daher aus der durch die Federn und Permanentmagnete gespeicherten potentiellen Energie.

Besonders bevorzugt werden im Rahmen der Bestimmung der potentiellen Energie auch externe Kräfte, welche im Rahmen seiner konkreten Verwendung auf den bistabilen Hubmagneten wirken, berücksichtigt. Dies kann beispielsweise die Gravitationskraft sein, wenn der Hubmagnet entgegen der Gravitationskraft ein Element anhebt. Alternativ oder zusätzlich kann es sich auch um externe Federkräfte handeln, beispielsweise wenn der Hubmagnet zum Bewegen eines federbelasteten Elementes eingesetzt wird.

Durch die in den beiden Hubendlagen ähnlich große potentielle Energie ergibt sich ein besonders energiesparender Betrieb des Hubmagneten. Im Ausführungsbeispiel wird die ähnlich große potentielle Energie insbesondere dadurch erreicht, dass die Feder mit der größeren Kraft und/oder Federrate den kleineren Federweg aufweist.

In dem in Fig. 2 gezeigten Ausführungsbeispiel des bistabilen Hubmagneten ist weiterhin ein zweiter, von der obigen Aspekten und insbesondere der unterschiedlichen Ausgestaltung der Federn unabhängiger Aspekt der vorliegenden Erfindung verwirklicht, und zwar durch die konstruktive Ausgestaltung des Stators, des Ankers sowie die Anordnung der Permanentmagnete und Spulen. Der Stator ist im Ausführungsbeispiel durch eine weichmagnetische Hülse 15 sowie die beiden Stirnabschnitte 20 und 30 gebildet, welche gemeinsam ein Gehäuse bilden, in dessen Inneren der weichmagnetische Anker 40 verschieblich angeordnet ist. Die Hülse 15 erstreckt sich zwischen dem ersten Stirnabschnitt 20 und dem zweiten Stirnabschnitt 30 über die gesamte Länge des Hubmagneten. Zwischen der ersten Seite des Ankers 40 und dem ersten Stirnabstand 20 ist ein erster Arbeitsluftspalt, zwischen der zweiten Seite des Ankers 40 und dem zweiten Stirnabschnitt 30 ein zweiter Arbeitsluftspalt gebildet.

Gemäß dem zweiten Aspekt sind zwei Permanentmagneten PM1 und PM2 vorgesehen, welche den Anker 40 entgegen der Kraft des Federsystems in den jeweiligen Hubendlagen halten. Die beiden Permanentmagnete PM1 und PM2 sind jeweils so zwischen der magnetischen Hülse 15 und dem jeweiligen Stirnabschnitt 20 bzw. 30 angeordnet, dass sie diese unter eine magnetische Spannung setzen. Hierzu können PM1 und PM2 beispielsweise aus jeweils einem oder mehreren radial polarisierten hartmagnetischen Ringen, bevorzugt NdFeB, gebildet werden. Alternativ können PM1 und PM2 aus radial oder diametral polarisierten, hartmagnetischen Ringsegmenten gebildet werden. Der Anker 40 schließt in der jeweiligen Hubendlage die Hülse 15 mit dem jeweiligen Stirnabschnitt 20 bzw. 30 über den Magnetkreisabschnitt 18, welcher als Rückschluss fungiert, magnetisch kurz, so dass der jeweilige Permanentmagnet in der jeweiligen Hubendlage eine Haltekraft auf den Anker 40 ausübt. Den beiden Hubendlagen ist jeweils eine Spule L1 bzw. L2 zugordnet, durch deren Bestromung der Anker aus der jeweiligen Hubendlage gelöst bzw. bei umgekehrter Stromrichtung entgegen der Kraft der jeweiligen Feder in seine Hubendlage gezogen werden kann.

Der in der ersten bzw. zweiten Hubendlage durch die Hülse, den Anker, den jeweiligen Stirnabschnitt und jeweiligen Permanentmagneten gebildete magnetische Teilkreis umgibt die jeweilige Spule L1 bzw. L2, so dass eine Bestromung der Spule in einer Stromrichtung der magnetischen Haltekraft des jeweiligen Permanentmagneten entgegen wirkt und so für eine Auslenkung des Ankers aus der jeweiligen Hubendlage sorgt. Ist die Haltekraft des Permanentmagneten überwunden, trägt die jeweilige Feder erheblich zur Bewegung des Ankers bei.

Die Spulen L1 und L2 sind in axialer Richtung des Hubmagneten zwischen den beiden Permanentmagneten PM1 und PM2 angeordnet. Die Hülse 15 weist einen mittleren Magnetkreisabschnitt 18 auf, welcher zwischen den beiden Spulen L1 und L2 so angeordnet ist, dass er sowohl in der ersten Hubendlage als auch in der zweiten Hubendlage magnetisch an den Anker 40 koppelt. In axialer Richtung schließen an diesem Magnetkreisabschnitt 18 der Hülse 15 auf beiden Seiten jeweils die Spulen L1 bzw. L2 an, neben welchen dann in axialer Richtung weiter außen die jeweiligen Permanentmagnete PM1 und PM2 angeordnet sind. Der Magnetkreisabschnitt 18 wird im Ausführungsbeispiel durch eine nach innen auskragende Erhebung der Innenwand der Hülse 15 gebildet, während die Spulen L1 und L2 bzw. Permanentmagnete F1 und F2 in Nuten bzw. Aussparungen am Innenumfang der Hülse 15 angeordnet sind.

Im Ausführungsbeispiel sind die Permanentmagneten PM1 und PM2 jeweils zwischen der Hülse 15 und einem in die Hülse hineinragenden Teil des jeweiligen Stirnabschnittes 20 bzw. 30 angeordnet. Die Spulen L1 und L2 sind dagegen zumindest teilweise neben dem Bewegungsbereich des Ankers 40 angeordnet.

Durch die Verwendung der axial außen angeordneten Permanentmagneten PM1 und PM2 kann gegenüber anderen Bauformen die Baulänge des Hubmagneten verringert werden.

Im Ausführungsbeispiel ist der Hubmagnet rotationssymmetrisch um die Achse 50 ausgeführt.

Gemäß einem weiteren Aspekt der vorliegenden Erfindung weisen die weichmagnetischen Stirnabschnitte 20 und 30 des Stators jeweils einen Befestigungsbereich 21 bzw. 31 auf, mit welchem sie mit der Hülse 15 in Verbindung stehen. Dies hat konstruktiv erhebliche Vorteile, da hierdurch eine einfache und stabile Verbindung zwischen den Stirnabschnitten und der Hülse im Verbindungsbereich 19 ermöglicht wird.

Da der Befestigungsbereich 21 bzw. 31 sich jedoch in radialer Richtung über den ersten bzw. den zweiten Permanentmagneten PM1 bzw. PM2 erstreckt, stellt er einen eigentlich unerwünschten magnetischen Kurzschluss zwischen der Hülse und dem jeweiligen Stirnabschnitt her. Der Befestigungsbereich ist daher vorzugsweise so ausgestaltet, dass er durch den jeweiligen Permanentmagneten magnetisch komplett gesättigt ist. Bevorzugt beträgt der magnetische Fluss, welcher von der Hülse über den Befestigungsabschnitt fließt, maximal 50 % des magnetischen Flusses, welcher in der jeweiligen Hubendlage von der Hülse über den Anker zu dem jeweiligen Stirnabschnitt fließt, bevorzugt maximal 20%.

Der Befestigungsbereich 21 bzw. 31 ist plattenförmig, insbesondere ringplattenförmig ausgestaltet. Weiterhin kann der Befestigungsbereich Aussparungen aufweisen, um das weichmagnetische Material im Bereich des Befestigungsbereiches zu verringern. In einer möglichen Ausgestaltung kann der Befestigungsbereich 31 nach außen hin weniger Material aufweisen, beispielsweise indem er nach außen hin dünner ausgestaltet ist, um so eine möglichst gleichmäßige Sättigung in diesem Bereich zu bewirken.

Der erste und der zweite Aspekt sind im Ausführungsbeispiel kombiniert verwirklicht, d.h. der Hubmagnet weist einen konstruktiven Aufbau gemäß dem zweiten Aspekt sowie unterschiedliche Federn gemäß dem ersten Aspekt auf. Auch die übrigen oben beschriebenen Aspekte sind in Kombination verwirklicht.

Innerhalb der Beschränkungen der beigefügten Ansprüche, die die Erfindung definieren, kann jeder einzelne der oben beschriebenen Aspekte eines Hubmagneten gemäß der vorliegenden Erfindung jedoch auch unabhängig von den anderen Aspekten verwirklicht werden. Die zu den einzelnen Aspekten beschriebenen Merkmale bilden die vorliegende Erfindung daher jeweils auch unabhängig von den zu den anderen Aspekten beschriebenen Merkmalen weiter. Weiterhin können auch nur einige der Aspekte miteinander kombiniert werden, wobei die vorliegende Erfindung sämtliche Kombination der oben beschriebenen Aspekte umfasst.

Insbesondere kann der konstruktive Aufbau gemäß dem zweiten Aspekt auch mit identischen Federn und/oder identischen magnetischen Haltekräften eingesetzt werden.

Weiterhin kann die Ausgestaltung mit unterschiedlichen Federn und/oder unterschiedlichen magnetischen Haltekräften und/oder einer asymmetrische Raststelle auch bei einer anderen konstruktiven Ausgestaltung des Haltemagneten eingesetzt werden.

Beispielsweise könnte statt den beiden außen liegenden Permanentmagnete PM1 und PM2 ein einzelner im Bereich des Magnetkreisabschnitts 18 angeordneter Permanentmagnet eingesetzt werden, welcher in beiden Hubendlagen die Hülse 15 und den Anker 40 unter eine magnetische Spannung setzt.

Weiterhin sind auch andere konstruktive Ausgestaltungen des Stators denkbar, beispielsweise mit zwei getrennten weichmagnetischen Abschnitten, zwischen welchen zumindest Teile des Ankers angeordnet sind, bspw. in Form einer Ankerplatte. Weiterhin alternativ oder zusätzlich sind auch Ausgestaltungen mit außen liegenden Ankerplatten und/oder mit am Anker angeordneten Permanentmagneten denkbar.

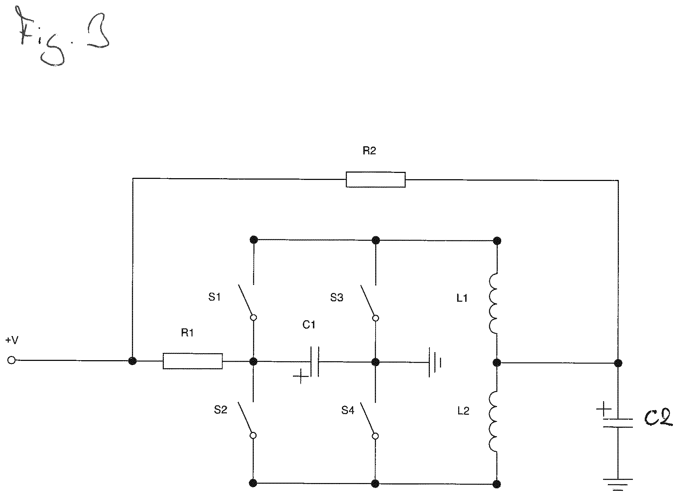

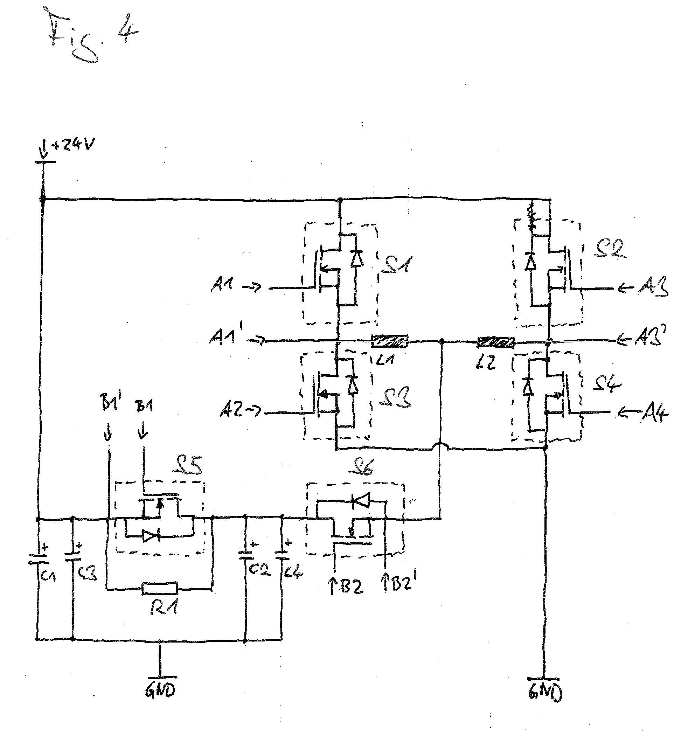

Mögliche Ausgestaltungen einer Steuerung zur Ansteuerung eines bistabilen Hubmagneten im Rahmen der vorliegenden Erfindung sind in Fig. 3 und 4 gezeigt. Sie können zur Ansteuerung beliebiger bistabiler Hubmagnete, welche mindestens zwei Spulen L1 und L2 aufweisen, eingesetzt werden. Besonders bevorzugt kommt die Steuerung bei bistabilen Hubmagneten zum Einsatz, bei welchen der Anker im unbestromten Fall permanentmagnetisch in der ersten und zweiten Hublage gehalten wird, wobei durch Bestromen der ersten Spule L1 und/oder der zweiten Spule L2 mit einer ersten Stromrichtung der Hubmagnet aus der ersten Hubendlage, und durch Bestromung der zweiten Spule L2 und/oder der ersten Spule L1 mit einer zweiten Stromrichtung der Anker aus der zweiten Hubendlage gelöst wird.

Besonders bevorzugt weist der Hubmagnet ein Federsystem mit einer ersten und einer zweiten Feder auf, wobei die erste Feder in der ersten Hubendlage auf den Anker eine Kraft in Richtung auf die Hubmittellage ausübt, und die zweite Feder in der zweiten Hubendlage eine Kraft auf den Anker in Richtung auf die Hubmittellage ausübt. Durch Bestromen mindestens der ersten Spule L1 mit einer zweiten Stromrichtung kann der Hubmagnet entgegen der Federkraft der ersten Feder in die erste Hubendlage gezogen werden, und durch Bestromung mindestens der zweiten Spule L2 mit einer zweiten Stromrichtung kann der Anker in die zweite Hubendlage gezogen werden.

Der Stator und der Anker können in der jeweiligen Hubendlage einen magnetischen Teilkreis bilden, welcher die jeweilige Spule L1 bzw. L2 umgibt, so dass ein Bestromen der jeweiligen Spule mit der ersten Stromrichtung die permanentmagnetische Haltekraft abschwächt.

Die Steuerungen können besonders bevorzugt zur Ansteuerung eines erfindungsgemäßen Hubmagneten eingesetzt werden, wie er oben beschrieben wurde, und besonderes bevorzugt zur Ansteuerung eines Hubmagneten, bei welchem ein oder mehrere der oben beschriebenen Aspekte verwirklicht sind. Weiterhin bevorzugt arbeiten die oben beschriebenen Hubmagnete gemäß der vorliegenden Erfindung so, wie dies soeben beschrieben wurde.

Beiden Ausführungsbeispielen der Steuerung ist gemeinsam, dass die Bestromung der Spulen L1 und L2 über ein oder mehrere Energiespeicher C1 , C2 erfolgt, welche über Schalter S1 bis S4 über die Spulen L1 und L2 entladen werden. Bei den Energiespeichern handelt es sich im Ausführungsbeispiel um Kondensatoren, insbesondere um Elektrolytkondensatoren. Hierzu wird im Ausführungsbeispiel eine durch die Schalter S1 bis S4 gebildete Vollbrücke eingesetzt, um die Richtung, in welcher die Entladung über die Spulen erfolgt, frei wählen zu können. Beiden Ausführungsbeispielen ist weiterhin gemein, dass mindestens ein erster Energiespeicher C1 über die in Reihe geschalteten Spulen L1 und L2 entladen werden kann. Mindestens ein zweiter Energiespeicher C2 kann dagegen über nur eine der beiden Spulen L1 oder L2 entladen werden. Hierfür kann der zweite Energiespeicher C2 mit dem Mittelabgriff zwischen den beiden Spulen L1 und L2 verbunden werden oder verbunden sein. Über welche der beiden Spulen L1 oder L2 die jeweilige Entladung erfolgt, wird über die Vollbrücke bestimmt, welche sowohl zur Ansteuerung der Entladungsrichtung des ersten Energiespeichers C1 , als auch zur Ansteuerung der Entladung des zweiten Energiespeichers C2 über die erste Spule L1 oder die zweite Spule L2 genutzt wird.

Bei dem in Fig. 3 gezeigten Ausführungsbeispiel steht der Energiespeicher C2 ständig mit dem Mittelabgriff zwischen den beiden Spulen in Verbindung. Wird daher die Entladung über die Vollbrücke freigeschaltet, entladen sich gleichzeitig der erste Energiespeicher in Reihe über die beiden Spulen L1 und L2, und der zweite Energiespeicher C2 über eine der beiden Spulen L1 und L2.

Bei dem in Fig. 4 gezeigten Ausführungsbeispiel steht der zweite Energiespeicher C2 dagegen schaltbar mit dem Mittelabgriff zwischen den beiden Spulen L1 und L2 in Verbindung, und zwar über den Schalter S6. Über einen weiteren Schalter S5 kann der zweite Energiespeicher C2 dagegen mit dem ersten Energiespeicher C1 parallel geschaltet werden.

Die Schaltung in Fig. 4 kann in einen ersten Betriebsmodus beide Energiespeicher C1 und C2 in Reihe über die beiden Spulen L1 und L2 entladen. In einem zweiten Betriebsmodus wird dagegen nur der erste Energiespeicher C1 in Reihe über die Spulen L1 und L2 entladen, der zweite Energiespeicher C2 dagegen über je eine der beiden Spulen L1 oder L2. Bevorzugt wird im zweiten Betriebsmodus der zweite Energiespeicher C2 mit einem zeitlichen Versatz zu der Vollbrücke geschaltet, d.h. der zweite Energiespeicher C2 wird erst mit dem Mittelabgriff zwischen den beiden Spulen verbunden, nachdem die Vollbrücke bereits eine Verbindung zwischen dem ersten Energiespeicher und den beiden Spulen hergestellt und den Stromkreis zur Entladung von C1 geschlossen hat. Bevorzugt wird der zweite Energiespeicher C2 jedoch so frühzeitig zugeschaltet, dass die Stellbewegung noch nicht eingesetzt hat.

Die Entladung des zweiten Energiespeichers C2 über den Mittelabgriff führt dazu, dass dieser nur über eine der beiden Spulen L1 oder L2 entladen wird. Zum einen steht hierdurch mehr Energie für diese Spule zur Verfügung. Als weiterer Vorteil ergibt sich, dass der Strom durch die andere Spule begrenzt wird und hierdurch eine Überkompensation vermieden wird.

Bevorzugt ist die Schaltung so ausgestaltet, dass der erste Betriebsmodus eingesetzt wird, um den Hubmagneten in eine erste Richtung zu bewegen, und der zweite Betriebsmodus, um den Hubmagneten in eine zweite Richtung zu bewegen. Insbesondere kann der erste Betriebsmodus, bei welchem die beiden Energiespeicher C1 und C2 parallel geschaltet sind und beide über die in Reihe geschalteten Spulen L1 und L2 entladen werden, für eine Bewegung von der ersten Hubendlage in die zweite Hubendlage eingesetzt werden, d. h. zum Ausfahren des Stifts. Für eine Bewegung von der zweiten Hubendlage in die erste Hubendlage, d.h. zum Einfahren des Stifts, kommt dagegen bevorzugt der zweite Betriebsmodus zum Einsatz, bei welchem der zweite Energiespeicher C2 parallel zu Energiespeicher C1 über eine der beiden Spulen L1 und L2 entladen wird, bevorzugt mit einem Zeitversatz gegenüber der Entladung des ersten Energiespeichers C1 . Eine solche unterschiedliche Ansteuerung der beiden Bewegungsrichtungen ist insbesondere dann von Vorteil, wenn der Hubmagnet eine asymmetrische Kennlinie und/oder unterschiedliche Federn aufweist.

Bevorzugt sind die Schalter der Vollbrücke sowie die Schalter zum Umschalten zwischen dem ersten und dem zweiten Betriebsmodus jeweils als Halbleiterschalter, insbesondere in Form eines MOSFET ausgebildet. In Fig. 4 ist dies gezeigt. Zum Ansteuern sind jeweils Steuereingänge A1 bis A4 und B1 und B2 vorgesehen, über welche eine Spannungsdifferenz gegenüber den Referenzanschlüssen A1 ', A3', B1 ' und B2' zur Ansteuerung der jeweiligen Schalters angelegt wird.

Weiterhin sind im Ausführungsbeispiel in Fig. 4 jeweils zwei erste Energiespeicher C1 und C3 und zwei zweite Energiespeicher C2 und C4 parallel geschaltet.

Bei dem in Fig. 3 gezeigten Ausführungsbeispiel erfolgt die Aufladung der Energiespeicher C1 und C2 über Wiederstände R1 und R2, mit welchen diese mit einer Spannungsversorgung +V in Verbindung stehen. Wird daher die Spannungsversorgung eingeschaltet, laden sich die Energiespeicher über die jeweiligen Widerstände.

Bevorzugt wird jedoch sowohl beim ersten, als auch beim zweiten Ausführungsbeispiel eine elektronisch geregelte Ladung der Energiespeicher vorgenommen, insbesondere mit einem konstanten Ladestrom.

Alternativ oder zusätzlich kann der Ladestrom, mit welchem die Energiespeicher geladen werden, einstellbar sein. Beispielsweise kann die Steuerung mehrere Betriebsmodi aufweisen, welche sich durch die Größe des Ladestroms unterscheiden, wobei die Steuerung bevorzugt zwischen den Betriebsmodi umschaltbar ist. Durch den Ladestrom wird die erforderliche Totzeit zwischen zwei Stellvorgängen wesentlich bestimmt. Bein einem hohen Ladestrom wird die Zeit, welche zwischen zwei Stellvorgängen notwendig ist, verkürzt. Ein niedriger Ladestrom verlängert dagegen diese Zeit. Durch die unterschiedlichen Betriebsmodi kann der Hubmagnet beispielsweise dann, wenn längere Zeiten zwischen zwei Stellvorgängen zulässig sind, mit einer Energieversorgung mit geringerer Leistung betrieben werden, ohne diese zu überlasten. Unterschiedliche Ladeströme können bspw. durch unterschiedliche Widerstände, oder durch eine entsprechende elektronische Steuerung verwirklicht werden, bevorzugt durch Schaltregler, beispielsweise Aufwärts- oder Abwärtswandler.

Auch unabhängig von der konkreten Ausgestaltung der Steuerung, wie sie oben beschrieben wurde, wird der Hubmagnet gemäß einem weiteren Aspekt der vorliegenden Erfindung so angesteuert, dass bei einem Abschalten der Spannungsversorgung der Hubmagnet von der ersten in die zweite Hubendlage bewegt wird. Bei einem Zuschalten der Versorgungsspannung wird der Hubmagnet dagegen zurück von der zweiten Hubendlage in die erste Hubendlage bewegt.

Bevorzugt wird eine Überwachung der Versorgungsspannung vorgenommen. Beispielsweise kann ein Abfallen der Versorgungsspannung mittels einer Flankenerkennung erkannt werden. Fällt die Versorgungsspannung ab, werden die Energiespeicher über die Spule oder Spulen des Hubmagneten entladen, um den Hubmagneten von der ersten in die zweite Hubendlage zu bewegen.

Bevorzugt werden nach dem Zuschalten der Versorgungsspannung zunächst die elektrischen Energiespeicher aufgeladen, wobei die Steuerung das Erreichen einer bestimmten Schwellspannung am Energiespeicher erkennt und daraufhin den Energiespeicher über die Spule oder Spulen des Hubmagneten so entlädt, dass dieser sich von der zweiten in die erste Hubendlage bewegt.

Eine solche Ausgestaltung hat den Vorteil, dass der erfindungsgemäße Hubmagnet problemlos zum Ersatz von monostabilen Hubmagneten eingesetzt werden kann.

Weist der Hubmagnet wie oben beschrieben eine gegenüber der Hubmittellage versetzte Raststelle auf, wird ein solcher Betrieb besonders sicher. Denn selbst dann, wenn die Versorgungsspannung ungewollt sehr kurz nach einem Schaltvorgang, mit welchem der Hubmagnet in die erste Hubendlage verfahren wurde, ausfällt, oder sonstige Probleme an den Energiespeichern auftreten, ist ein Verfahren in die Raststelle immer noch möglich, da hierfür nur sehr wenig Energie benötigt wird. In dieser Raststelle ist der Hubmagnet jedoch bereits weitgehend zur zweiten Hubendlage hin ausgefahren.

Hierdurch wird die Sicherheit beim Einsatz eines bistabilen Hubmagneten zum Ein-und Ausfahren des Stifts erheblich erhöht.

## Patentansprüche

1. Elektromagnetisches Verriegelungselement zum Verriegeln des Gelenkes einer Gelenk-Orthese oder Gelenk-Prothese, insbesondere einer Knie- Orthese oder Knie-Prothese, umfassend:
- einen Hubmagneten (10) mit mindestens einer Spule (L1, L2) und mindestens einem Permanentmagneten (PM1, PM2);
- einen Stifts (6), welcher mit Hilfe des Hubmagneten (10) eingefahren werden kann, zum Verriegeln des Gelenkes,
- einen oder mehrere elektrische Energiespeicher (C1, C2, C3, C4), insbesondere Kondensatoren,
- eine elektrische Schaltung, welche mit Hilfe von Halbleiterschaltern (11) den oder die Energiespeicher (C1, C2, C3, C4) über die mindestens eine Spule (L1, L2) des Hubmagneten (10) so entlädt, dass der Stift (6) ausgefahren wird, **dadurch gekennzeichnet, dass** der Hubmagnet (10) bistabil zum Einfahren und Ausfahren des Stiftes (6) ausgebildet ist und das Verriegelungselement ein Federsystem mit einer ersten Feder (F1) aufweist, welche in einer ersten Hubendlage auf den oder die Anker (40) des Hubmagneten (10) eine Kraft in Richtung die Hubmittellage ausübt, sowie eine zweiten Feder (F2) aufweist, welche in einer zweiten Hubendlage auf den oder die Anker (40) eine Kraft in Richtung die Hubmittellage ausübt, wobei der oder die Anker (40) im stromlosen Fall in beiden Hubendlagen entgegen der Federkraft permanentmagnetisch gehalten werden, wobei die erste und die zweite Feder (F1, F2) unterschiedlich lange Federwege aufweisen
und/oder
in der jeweiligen Hubendlage unterschiedlich große Kräfte auf den oder die Anker (40) ausüben und/oder unterschiedlich große Federraten aufweisen.

2. Verriegelungselement nach Anspruch 1, wobei sich die in den beiden Hubendlagen im Verriegelungselement gespeicherte potentielle Energie ausschließlich der elektrischen Energie und im unbestromten Fall nicht um mehr als 50% des größeren Wertes voneinander unterscheidet, bevorzugt um nicht mehr als 25%.

3. Verriegelungselement nach einem der vorangegangenen Ansprüche, wobei der bistabile Hubmagnet (10) eine asymmetrische Kennlinie aufweist, und/oder wobei die magnetische Haltekraft des Hubmagneten (10) in derjenigen Hubendlage am höchsten ist, in welcher sich der Stift (6) des Verriegelungselements in seiner ausgefahrenen Position befindet und/oder wobei die magnetische Haltekraft in einer der Hubendlagen zwischen 20% und 80% der magnetischen Haltekraft in der anderen Hubendlage beträgt, bevorzugt zwischen 30% und 70%.

4. Verriegelungselement nach einem der vorangegangenen Ansprüche, wobei das Verriegelungselement im unbestromten Fall eine Raststelle in einer Position aufweist, in welcher der Stift (6) teilweise ausgefahren ist.

5. Verriegelungselement nach einem der vorangegangenen Ansprüche, wobei die Steuerung eine Unterbrechung und/oder ein Abschalten der Spannungsversorgung des Verriegelungselements erkennt und in Reaktion hierauf den Stift (6) ausfährt.

6. Verriegelungselement nach einem der vorangegangenen Ansprüche, wobei die Steuerung so ausgestaltet ist, dass auf ein Zuschalten der Versorgungsspannung hin der oder die elektrischen Energiespeicher (C1, C2, C3, C4), vorzugsweise Kondensatoren, aufgeladen werden und dass das Erreichen einer bestimmten Schwellspannung am elektrischen Energiespeicher (C1, C2, C3, C4) von der Steuerung erkannt wird, woraufhin selbige den oder die Energiespeicher (C1, C2, C3, C4) über den bistabilen Hubmagneten (10) so entlädt, dass der Stift (6) eingefahren wird.

7. Verriegelungselement nach einem der vorangegangenen Ansprüche, wobei der bistabile Hubmagnet (10) über eine Vollbrücke angesteuert wird.

8. Verriegelungselement nach einem der vorangegangenen Ansprüche, mit einer Steuerung mit mindestens einem ersten und einem zweiten elektrischem Energiespeicher (C1, C2, C3, C4), wobei der erste Energiespeicher (C1, C3) in Serie über zwei Spulen (L1, L2) des Hubmagneten (10) entladbar ist, und wobei der zweite Energiespeicher (C2, C4) über eine der zwei Spulen (L1, L2) des Hubmagneten (10) entladbar ist.

9. Verriegelungselement nach einem der vorangegangenen Ansprüche, mit Mitteln zur Positionserfassung des Verriegelungselements und/oder mit Mitteln zur Erfassung der Winkelstellung des Gelenkes, und/oder wobei der Stift (6) einen Dämpfungsmechanismus aufweist und/oder mit einem solchen verbunden ist, und/oder wobei die Energiespeicher (C1, C2, C3, C4) über eine Batterie und/oder einen Akkumulator geladen werden.

10. Verriegelungselement nach einem der vorangegangenen Ansprüche, wobei die zweite Hubendlage der komplett ausgefahrenen Position des Stifts (6) entspricht und die erste Hubendlage der komplett eingefahrenen Position.

11. Verriegelungselement nach einem der vorangegangenen Ansprüche, wobei der Stator und der oder die Anker (40) in einer der Hubendlagen und bevorzugt in der ersten Hubendlage eine geometrische Kennlinienbeeinflussung aufweisen, und/oder
wobei sich die Differenz zwischen dem Betrag der magnetischen Haltekraft und dem Betrag der Kraft, welche die jeweilige Feder (F1, F2) aufbringt, in den beiden Hubendlagen um maximal 50% des größeren Wertes unterscheidet.

12. Verriegelungselement nach einem der vorangegangenen Ansprüche, wobei der Hubmagnet (10) einen Stator aufweist,
wobei der Stator eine weichmagnetische Hülse (15) und einen ersten und zweiten weichmagnetischen Stirnabschnitt aufweist, welche ein Gehäuse bilden, in welchem der Anker (40) verschieblich angeordnet ist,
wobei zwischen dem Anker (40) und dem ersten Stirnabschnitt (20) mindestens ein erster Arbeitsluftspalt und zwischen dem Anker (40) und dem zweiten Stirnabschnitt (30) mindestens ein zweiter Arbeitsluftspalt vorgesehen ist;
wobei am Stator der mindestens eine Permanentmagnet (PM1, PM2) und die mindestens eine erste und eine zweite Spule (L1, L2) angeordnet sind, wobei der Anker (40) in der ersten Hubendlage mit der Hülse (15) und dem ersten Stirnabschnitt (20) einen ersten magnetischen Teilkreis bildet, welcher zumindest die erste Spule (L1) umgibt, während der oder die Arbeitsluftspalte mit dem zweiten Stirnabschnitt (30) maximal geöffnet sind;
und wobei der Anker (40) in der zweiten Hubendlage mit der Hülse (15) und dem zweiten Stirnabschnitt (30) einen zweiten magnetischen Teilkreis bildet, welcher zumindest die zweite Spule (L2) umgibt, während der oder die Arbeitsluftspalte mit dem ersten Stirnabschnitt (20) maximal geöffnet sind,
wobei bevorzugt mindestens ein erster und ein zweiter Permanentmagnet (PM1, PM2) vorgesehen sind, wobei die erste und die zweite Spule (L1, L2) in axialer Richtung zwischen dem ersten und dem zweiten Permanentmagneten (PM1, PM2) angeordnet sind,
wobei der erste Permanentmagnet (PM1, PM2) die Hülse (15) und den ersten Stirnabschnitt (20) und der zweite Permanentmagnet (PM1, PM2) die Hülse (15) und den zweiten Stirnabschnitt (30) unter eine magnetische Spannung setzen.

13. Gelenk-Orthese oder Gelenk-Prothese, insbesondere Knie-Orthese oder Knie-Prothese, mit einem ersten Element und einem zweiten Element, welche über ein Drehgelenk schwenkbar miteinander verbunden sind, und mit einem elektromagnetischen Verriegelungselement nach einem der vorangegangenen Ansprüche zum Verriegeln des Drehgelenkes, insbesondere zum Verriegeln des Drehgelenkes in einer gestreckten Position der Gelenk-Orthese bzw. Gelenk-Prothese.

## Claims

1. An electromagnetic locking element for locking a joint orthosis or joint prosthesis, in particular a knee orthosis or knee prosthesis, comprising:
- a solenoid (10) with at least one coil (L1, L2) and at least one permanent magnet (PM1, PM2);
- a pin (6), which can be retracted and extended with the aid of the solenoid (10) for locking the joint,
- one or several electrical energy stores (C1, C2, C3, C4), in particular capacitors,
- an electrical control system, which, with the aid of semiconductor switches (11), discharges the energy store or stores (C1, C2, C3, C4) via the at least one coil (L1, L2) of the solenoid (10) in such a way that the pin (6) is extended, **characterized in that** the solenoid (10) is designed to be bistable for retracting and extending the pin (6), and that the locking has a spring system with a first spring (F1), which exerts a force on the armature (40) or armatures (40) of the solenoid (10) towards the central stroke position when in a first stroke end position, and a second spring (F2), which exerts a force on the armature (40) or armatures (40) towards the central stroke position when in a second stroke end position, wherein the armature (40) or armatures (40) is/are held, permanently magnetized, against the spring force in both stroke end positions when no current is present, wherein the spring deflections of the first and second springs (F1, F2) are preferably different in length and/or the first and second springs (F1, F2) exert forces of different strength on the armature (40) or armatures (40) in the respective stroke end position and/or have spring rates of different size.

2. The locking element according to claim 1, wherein the amounts of potential energy stored in the locking element in the two stroke end positions, excluding the electrical energy and when no current is present, do not differ from one another by more than 50 % of the greater value, preferably by no more than 25 %.

3. The locking element according to one of the above claims, wherein the bistable solenoid (10) has an asymmetrical characteristic line and/or wherein the magnetic holding force of the solenoid (10) is highest in the end position in which the pin of the locking element is extended, which is preferably reached by way of a geometric characteristic line modifier and/or wherein the magnetic holding force in one of the end positions is between 20 % and 80 % of the magnetic holding force in the other end position, preferably between 30 % and 70 %.

4. The locking element according to one of the above claims, wherein the locking element has a resting point at a position in which, when no current is present, the pin (6) is partially extended.

5. The locking element according to one of the above claims, wherein the control system recognizes an interruption and/or switching off of the power supply of the locking element and in response to this, extends the pin (6), wherein the failure of the supply voltage is preferably recognized by means of edge detection.

6. The locking element according to one of the above claims, wherein the control system is configured such that, in response to a switching on of the supply voltage, the electrical energy store or stores (C1, C2, C3, C4), preferably capacitors, is/are charged, and that the control system recognizes when a certain threshold voltage is reached in the electrical energy store (C1, C2, C3, C4); in response to this, said control system discharges the energy store or stores (C1, C2, C3, C4) via the bistable solenoid (10) in such a way that the pin (6) is retracted.

7. The locking element according to one of the above claims, wherein the bistable solenoid (10) is controlled via a full bridge.

8. The locking element according to one of the above claims, with a control system with at least a first and a second electrical energy store (C1, C2, C3, C4), wherein the first energy store (C1, C3) can be serially discharged via two coils (L1, L2) of the solenoid (10), and wherein the second energy store (C2, C4) can be discharged via one of the two coils (L1, L2) of the solenoid (10).

9. The locking element according to one of the above claims, with an instrument for measuring the position of the locking element and/or an instrument for measuring the angular position of the joint and/or wherein the pin comprises a damping mechanism and/or is connected to such a mechanism,
and/or wherein the energy store (C1, C2, C3, C4) is charged via a battery and/or a rechargeable battery.

10. The locking element according to one of the above claims
wherein the second stroke end position represents the completely extended position of the pin (6) and the first end position the completely retracted position

11. The locking element according to one of the above claims, wherein the stator and the armature or armatures (40) have a geometric characteristic line modifier in one of the stroke end positions and preferably in the first stroke end position,
wherein the difference between the value of the magnetic holding force and the value of the force applied by the respective spring (F1, F2) in both stroke end positions differs by a maximum of 50 % of the greater value.

12. The locking element according to one of the above claims, wherein the locking element comprises a stator,
wherein the stator feature a magnetically soft casing (15), and a first and second magnetically soft front section, which form a housing in which the armature (40) is arranged such that it can be displaced,
wherein at least a first working air gap is provided between the armature (40) and the first front section (20), and the at least a second working air gap between the armature (40) and the second front section (30);
wherein the at least permanent magnet and at least a first and a second coil (L1, L2) are arranged on the stator,
wherein, when in the first stroke end position, the armature (40) forms a first port-magnetic-circuit with the casing (15) and the first front section (20), said port-magnetic-circuit surrounding at least the first coil (L1), while the working air gap or working air gaps with the second front section (30) are opened to a maximum degree;
and wherein, when in the second stroke end position, the armature (40) forms a second port-magnetic-circuit with the casing (15) and the second front section (30), said port-magnetic-circuit surrounding at least the second coil (L2), while the working air gap or working air gaps with the first front section (20) are opened to a maximum degree,
wherein preferably at least a first and a second permanent magnet (PM1, PM2) are provided, wherein the first and the second coil (L1, L2) are arranged in the axial direction between the first and the second permanent magnet (PM1, PM2), wherein the first permanent magnet (PM1, PM2) applies a magnetic voltage to the casing (15) and the first front section (20) and the second permanent magnet (PM1, PM2) applies a magnetic voltage to the casing (15) and the second front section (30).

13. A joint orthosis or joint prosthesis, especially a knee orthosis or knee prosthesis, with a first element and a second element that are connected to one another via a swivel joint such that they can be swivelled and with an electromagnetic locking element according to one of the above claims for locking the swivel joint, especially for locking the swivel joint in an extended position or the joint orthosis or joint prosthesis.

## Revendications

1. Elément de verrouillage électromagnétique pour verrouiller l'articulation d'une orthèse d'articulation ou d'une prothèse d'articulation, en particulier d'une orthèse de genou ou d'une prothèse de genou, comprenant :
- un aimant de levage (10) présentant au moins une bobine (L1, L2) et au moins un aimant permanent (PM1, PM2) ;
- une tige (6) qui peut être rétractée à l'aide de l'aimant de levage (10), pour verrouiller l'articulation,
- un ou plusieurs dispositifs électriques de stockage d'énergie (C1, C2, C3, C4), en particulier des condensateurs,
- un circuit électrique qui, à l'aide de commutateurs à semi-conducteurs (11), fait décharger le ou les dispositifs de stockage d'énergie (C1, C2, C3, C4) par l'intermédiaire de ladite au moins une bobine (L1, L2) de l'aimant de levage (10), de telle sorte que la tige (6) est déployée,
**caractérisé en ce que**
l'aimant de levage (10) est réalisé bistable pour rétracter et déployer la tige (6), et l'élément de verrouillage comprend un système de ressorts ayant un premier ressort (F1) qui, dans une première position de fin de course, exerce une force en direction de la position centrale de la course sur le ou les induits (40) de l'aimant de levage (10), et un deuxième ressort (F2) qui, dans une deuxième position de fin de course, exerce une force en direction de la position centrale de la course sur le ou les induits (40), le ou les induits (40) étant retenus dans les deux positions de fin de course par voie magnétique permanente à l'encontre de la force des ressorts, dans le cas non alimenté, et les premier et deuxième ressorts (F1, F2) présentant des débattements de différentes longueurs et/ou exerçant, dans la position de fin de course respective, des forces de différentes intensités sur le ou les induits (40) et/ou présentant différentes constantes de rappel.

2. Elément de verrouillage selon la revendication 1,
dans lequel l'énergie potentielle stockée dans l'élément de verrouillage dans les deux positions de fin de course, à l'exclusion de l'énergie électrique et dans le cas non alimenté, ne diffère pas de plus de 50%, de préférence pas de plus de 25%, de la plus grande valeur.

3. Elément de verrouillage selon l'une des revendications précédentes,
dans lequel l'aimant de levage bistable (10) présente une caractéristique asymétrique, et/ou la force de retenue magnétique de l'aimant de levage (10) est la plus élevée dans la position de fin de course dans laquelle la tige (6) de l'élément de verrouillage se trouve dans sa position déployée, et/ou la force de retenue magnétique dans l'une des positions de fin de course est comprise entre 20% et 80%, de préférence entre 30% et 70%, de la force de retenue magnétique dans l'autre position de fin de course.

4. Elément de verrouillage selon l'une des revendications précédentes, l'élément de verrouillage présentant, dans le cas non alimenté, un point d'encliquetage dans une position dans laquelle la tige (6) est partiellement déployée.

5. Elément de verrouillage selon l'une des revendications précédentes,
dans lequel la commande reconnaît une interruption et/ou une coupure de l'alimentation en tension de l'élément de verrouillage et, en réponse à cela, fait déployer la tige (6).

6. Elément de verrouillage selon l'une des revendications précédentes,
dans lequel la commande est conçue de telle sorte que suite à une mise en circuit de la tension d'alimentation, le ou les dispositifs électriques de stockage d'énergie (C1, C2, C3, C4), de préférence des condensateurs, sont chargés, et que la commande reconnaît le franchissement d'une tension de seuil déterminée au niveau du dispositif électrique de stockage d'énergie (C1, C2, C3, C4), suite à quoi celle-ci fait décharger le ou les dispositifs de stockage d'énergie (C1, C2, C3, C4) par l'intermédiaire de l'aimant de levage bistable (10), de telle sorte que la tige (6) est rétractée.

7. Elément de verrouillage selon l'une des revendications précédentes,
dans lequel l'aimant de levage bistable (10) est piloté par l'intermédiaire d'un pont intégral.

8. Elément de verrouillage selon l'une des revendications précédentes, comprenant une commande ayant au moins un premier et un deuxième dispositif électrique de stockage d'énergie (C1, C2, C3, C4), le premier dispositif de stockage d'énergie (C1, C3) pouvant être déchargé en série par deux bobines (L1, L2) de l'aimant de levage (10), et le deuxième dispositif de stockage d'énergie (C2, C4) pouvant être déchargé par l'une des deux bobines (L1, L2) de l'aimant de levage (10).

9. Elément de verrouillage selon l'une des revendications précédentes, comprenant des moyens de détection de la position de l'élément de verrouillage et/ou comprenant des moyens de détection de la position angulaire de l'articulation, et/ou
la tige (6) comprenant un mécanisme d'amortissement et/ou étant reliée à un tel mécanisme, et/ou le dispositif de stockage d'énergie (C1, C2, C3, C4) étant chargé par une batterie et/ou par un accumulateur.

10. Elément de verrouillage selon l'une des revendications précédentes,
dans lequel
la deuxième position de fin de course correspond à la position complètement déployée de la tige (6), et la première position de fin de course correspond à la position complètement rétractée.

11. Elément de verrouillage selon l'une des revendications précédentes,
dans lequel le stator et le ou les induits (40) présentent, dans l'une des positions de fin de course et de préférence dans la première position de fin de course, une influence géométrique sur la courbe caractéristique, et/ou
la différence entre la valeur de la force de retenue magnétique et la valeur de la force exercée par le ressort respectif (F1, F) diffère au maximum de 50% de la plus grande valeur, dans les deux positions de fin de course.

12. Elément de verrouillage selon l'une des revendications précédentes,
dans lequel l'aimant de levage (10) présente un stator,
le stator présente une douille magnétique douce (15) et une première et une deuxième portion frontale magnétique douce, qui forment un boîtier dans lequel l'induit (40) est disposé de façon mobile en translation,
au moins un premier entrefer de travail est prévu entre l'induit (40) et la première portion frontale (20), et au moins un deuxième entrefer de travail est prévu entre l'induit (40) et la deuxième portion frontale (30) ;
au moins un aimant permanent (PM1, PM2) et ladite au moins une première et une deuxième bobine (L1, L2) sont disposés sur le stator,
dans la première position de fin de course, l'induit (40) forme, avec la douille (15) et la première portion frontale (20), un premier circuit magnétique partiel qui entoure au moins la première bobine (L1), tandis que le ou les entrefers de travail sont ouverts au maximum, avec la deuxième portion frontale (30) ;
et, dans la deuxième position de fin de course, l'induit (40) forme, avec la douille (15) et la deuxième portion frontale (30), un deuxième circuit magnétique partiel qui entoure au moins la deuxième bobine (L2), tandis que le ou les entrefers de travail sont ouverts au maximum, avec la première portion frontale (20),
de préférence, il est prévu au moins un premier et un deuxième aimant permanent (PM1, PM2), les première et deuxième bobines (L1, L2) étant disposées dans la direction axiale entre les premier et deuxième aimants permanents (PM1, PM2),
le premier aimant permanent (PM1, PM2) met la douille (15) et la première portion frontale (20) sous une tension magnétique, et le deuxième aimant permanent (PM1, PM2) met la douille (15) et la deuxième portion frontale (30) sous une tension magnétique.

13. Orthèse d'articulation ou prothèse d'articulation, en particulier orthèse de genou ou prothèse de genou, comprenant un premier élément et un deuxième élément qui sont reliés l'un à l'autre de façon mobile en pivotement par une articulation rotative, et comprenant un élément de verrouillage électromagnétique selon l'une des revendications précédentes pour verrouiller l'articulation rotative, en particulier pour verrouiller l'articulation rotative dans une position étirée de l'orthèse d'articulation ou de la prothèse d' articu lation.
